(19) Europäisches Patentamt

European Patent Office

Office européen des brevets

(11) **EP 0 970 655 A1**

(12) **EUROPEAN PATENT APPLICATION**
published in accordance with Art. 158(3) EPC

(43) Date of publication:
**12.01.2000 Bulletin 2000/02**

(51) Int. Cl.[7]: **A61B 5/00**

(21) Application number: **98900541.8**

(86) International application number:
**PCT/ES98/00011**

(22) Date of filing: **20.01.1998**

(87) International publication number:
**WO 98/31275 (23.07.1998 Gazette 1998/29)**

(84) Designated Contracting States:
**AT BE CH DE DK ES FI FR GB GR IE IT LI LU MC NL PT SE**
Designated Extension States:
**AL LT LV MK RO SI**

(30) Priority: **20.01.1997 ES 9700089**

(71) Applicant:
**Carpe Diem Comercial Sanitaria
28034 Madrid (ES)**

(72) Inventors:
• **ORDONEZ PEREZ, Alberto
E-28034 Madrid (ES)**
• **ZAMORA MENARGUEZ, Jose
E-28034 Madrid (ES)**

(74) Representative:
**Elzaburu Marquez, Alberto et al
Elzaburu S.A.,
Miguel Angel, 21
28010 Madrid (ES)**

(54) **APPARATUS AND SYSTEM FOR THE TELEMATIC CONTROL OF PHYSIOLOGICAL PARAMETERS OF PATIENTS**

(57) The portable and reduced size apparatus has an analyser for analysing the glucose in the blood, and providing a series of values. Said values can be processed in situ by a microprocessor and displayed on a screen of a personal computer connected to the glucose analyser. The telematic control system for patients provided with the apparatus of the invention would provide, simultaneously, that the data calculated in the portable apparatus would be transmitted to a central unit for the remote interpretation of the data through a mobile telephone which is also interconnected with the microprocessor and the analyser. Said transmission may be a direct bidirectional transmission (central unit-patient et vice versa) or a multidirectional transmission (central unit-peripheral units-patient) with as many connections as desired.

Printed by Xerox (UK) Business Services
2.16.7/3.6

## Description

### Field of the Invention

The invention belongs to the field of measurement of certain physiological parameters, specifically the blood glucose levels, carried out by patients themselves, and processing thereof in a remote unit.

### State of the Art

At first glucose analysis in diabetics was carried out indirectly, by quantifying glucose in the urine. This method of analysis represented a great advance in the diagnosis and monitoring of the disease. However, this method did not serve to detect the hypoglycemias nor either the instantaneous real values that patients had at the time of analysis, for which reason the results were merely guiding. Nowadays, these processes are limited to very specific circumstances, such as, for example, the quantification of ketone bodies.

The next step in the control of diabetes was the measurement of glucose in capillary blood. The first autoanalyzers appeared in the United States, developed by the Miles company, Spanish patents ES384417 and ES466707 (the latter under the name of KDK) and replaced laboratory analyses. That first generation of autoanalyzers gave the blood glucose level in just one minute and by means of a blood sample obtained by a simple prick in a finger using reactive strips. However, their high cost at that time placed them out of the reach of many patients. With the passage of time, the price of autoanalyzers has fallen considerably, which has made their use practically universal.

This universalization of the use of autoanalyzers entailed an immediate advantage, which is the radical reduction of consultations in hospitals and health centres to carry out glucose tests; and also a decrease in the number of admissions of diabetics. Moreover, the use of autoanalyzers has shown that self-monitoring is beginning to be a determining factor in the decrease in hospital admissions, which frees the health system from an economic burden that more than compensates for the cost of the reactive strips necessary for the controls.

The evolution of the autoanalyzers has not only meant an improvement in their accuracy and cost, but also in their ease of use. The first autoanalyzers were large devices that required connection to the electricity mains, complex calibration and cumbersome handling, while current apparatus are no larger than a credit card, and are autonomous and extraordinarily easy to use. The reactive strips have also evolved substantially: the first ones needed to be washed and dried, while current ones may be read directly, without the patient having to carry out any manipulation with them. Likewise, the reactive strips have increased their reading range, reaching 0 - 600 mg/dl in the case of some brands, and the physico-chemical principles of control have improved in terms of greater accuracy, speed and economy.

Diabetes is a chronic disease that brings with it severe physical and psychological harm to patients and very high economic costs for public and private health systems. This harm and these costs will increase as more cases are diagnosed, as life expectancy increases and as the demand for health care grows on the part of the affected groups.

Self-analysis of glycemia has revolutionised care for diabetic patients. Without doubt, during the last decade self-analysis of glycemia achieved general acceptance both among health care workers and patients. It is estimated that one million diabetics now use glycemia analysis devices at home. It is believed that long-term intensive therapy will have a clear impact upon certain diabetic complications, such as micro and macro vascular disorders. Strict control has already made it possible for diabetic women to have healthy babies of normal weight.

Control techniques may be grouped into two broad categories: techniques that evaluate the patient's medium-term situation and those that indicate instantaneously the blood glucose level.

For some time now, the fructosamine techniques and those of haemoglobin Alc quantification have been used in combination. The first method allows the quantification of metabolic control for short periods - one month -, while the second is useful for assessing patient control for the last three months.

Until now the use of these techniques involved carrying out a blood analysis. Nowadays, the multinational chemical Bayer Pharmaceutical Company has developed a quantification system of glycosylated haemoglobin Alc that is carried out, in the specialist's surgery itself using a simple table-top apparatus without having to send the blood sample to the laboratory. The considerable success of this technique, with its low cost and high degree of accuracy, allows the specialist to immediately evaluate metabolic control and avoids travelling and repeated consultations on the part of the patient. In the same sense, Bayer has developed a similar process for the quantification of renal damage by means of the determination of microalbuminuria.

## INSTANTANEOUS TECHNIQUES

These techniques provide immediate information regarding blood glucose levels. Different laboratories offer autonomous analysis devices whose easy operation allows them to be used by the patient.

## METHOD OF ANALYSIS

The test methods for measuring blood sugar are generally divided into three categories: reduction, calorimetric and enzymatic methods. Reduction methods involve the reduction of a metallic compound by carbohydrates. Owing to its lack of specificity to glucose, this method is liable to yield false positives and, currently, it is rarely used. The calorimetric methods, such as the reaction with o-toluidine, react with other monosaccharide aldehydes and are not specific to glucose. Therefore, the enzymatic methods are preferred for glycemia testing. Most laboratories use systems with reagents based on enzymes, either hexokinase or glucose oxidase, which are very specific to glucose. All the glucose self-analysis systems available on the market use glucose oxidase technology.

ANALYSIS SAMPLE: WHOLE BLOOD, PLASMA OR SERUM.

The type of blood sample suitable for glycemia testing varies depending on the method used. Glycemia self-analysis systems use only whole blood samples. Some laboratory systems measure the glucose in whole blood, plasma or serum, while others can only do this in serum or plasma.

Plasma and serum have a slightly different glucose concentration to that of whole blood. Although glucose is uniformly distributed in the liquid phase of blood, red blood cells have a solid phase that does not contain glucose. In whole blood, the solid phase of red blood cells dilutes the concentration of glucose in plasma.

SOURCE OF THE SAMPLE: ARTERIAL, CAPILLARY, VENOUS

Arterial blood has higher levels of glycemia, followed by capillary blood and venous blood. Before meals, arterial glycemia levels are approximately 5 mg/dl higher than capillary levels. Capillary levels are from 2 to 5 mg/dl higher than venous levels. After meals, glycemia levels in arterial and capillary blood may be from 20 to 70 mg/dl higher than in venous blood.

When the result of self-analysis of capillary blood sugar obtained by pricking a finger is compared with a laboratory result, the possible recent consumption of food must be taken into account. In a recent study in which 33 diabetic subjects took part (who were studied at different times after consuming food), the blood sugar measurements showed that most of the levels in whole capillary blood were only slightly higher than the levels in whole venous blood. However some patients had significantly higher capillary blood sugar levels (up to 48 mg/dl higher than the venous levels).

SAMPLING TIMES

The glycemia levels of diabetic patients may fluctuate significantly over short periods of time. Therefore, the time when samples are taken may affect the results of the blood sugar test when two different blood samples are extracted for comparison (eg. a sample by means of capillary pricking in the finger for a glycemia self-analysis test and a venous sample for a laboratory determination). Thus, samples must be extracted within a short period of time between one and the other.

ADDITIVES

There are several preservative or anticoagulant agents which, when added to a blood sample, preserve it and prevent its coagulation. A sample that is coagulated, although only partly, does not react suitably on a reactive strip and should not be used. Samples treated with anticoagulants of the heparin or ethylenediaminetetraacetic acid (EDTA) type are suitable for glycemia self-analysis tests. However, fluoride inhibits enzymatic reactions in tests with strips, which results in low blood sugar readings. It must be taken into account that samples treated with fluoride may be used in some laboratory methods with glucose oxidase.

GLYCOLYSIS

Blood samples must be subjected to glycemia analysis within 30 minutes after they are taken. With the glycemia self-analysis methods this does not generally pose a problem as the patient carries out the test immediately after pricking a finger. However, the glucose concentration in a whole blood sample to which no antiglycolytic agent has been added decreases at a rate of approximately 7 to 10 mg/dl per hour at room temperature. This decrease is due to glycolysis. Given that the decrease in glucose in whole blood is proportional to hematocrit levels, glycolysis is faster in blood samples that have higher hematocrit levels.

INTERFERING SUBSTANCES

The use of glucose oxidase for measurements with reactive strips ensures a high degree of specificity to glucose. Glucose oxidase (G0) acts on glucose to form gluconic acid and hydrogen peroxide ($H_2O_2$). In self-analysis methods based on spectrophotometry, the reduced chromogen (colourless) is oxidised by the hydrogen peroxide by means of the action of the peroxidase (PO) to form an oxidised chromogen (coloured). The amount of oxidised chromogen formed is proportional to the amount of glucose present in the sample. The natural oxidant or reductant substances such as uric acid or glutathione, may interfere with the reaction of peroxidase. Although there is no proof that the presence of normal physiological concentrations of these substances causes clinically significant errors, differences may be observed between patients. The drugs may interfere with the reaction of peroxidase and, in some cases, may cause significant errors in the test results.

The presence of high bilirubin levels may cause a decrease in the values of glycemia with some methods that use glucose oxidase. Levels of ascorbic acid that are very much above normal values may also cause a reduction in the results of glycemia when they interfere with the oxidation of chromogen.

USER'S TECHNIQUE AND QUALITY CONTROL

Analysis procedures vary with the different systems of glycemia self-analysis and the recommended procedures must be followed. The 1986 consensus statement on glycemia self-analysis concluded that all the systems were sufficiently accurate; however, the user technique is the main source of erroneous results. Therefore, quality controls must be carried out regularly. These controls consist of maintenance, calibration and verification of the system and checking of the control solution. The application of an adequate volume of blood sample to the reactive strip, starting the analysis at the right moment, controlling the reaction time and the elimination of blood, whether by drying or wiping, are steps of the procedure that are sensitive to error on the part of the user. Quality control and correct technique on the user's part are also important in clinical laboratory tests. The calibrators, controls, reagents, tubes, sampling apparatus and other specific parts of the instruments must be regularly checked. Laboratories have well-defined quality control procedures. The accuracy of laboratory glycemia tests is controlled regularly by means of external quality control programmes. In a test on 1,754 authorized laboratories, a sample with an average glucose concentration of 94 mg/dl produced an interval ((+/-)2 SD) of 76 to 122 mg/dl, and a sample with an average glucose concentration of 145 mg/dl produced an interval of 123 to 167 mg/dl. Thus, even in a rigorously controlled laboratory environment, in which trained technicians carry out the tests, there may be a variation between laboratories of up to (+/-) 19% in the results of blood sugar tests.

THE ONE TOUCH SYSTEM TECHNOLOGY

Until the introduction of the One Touch system all glycemia self-analysis apparatus on the market could be defined as "first generation" technology that evolved from reactive strips that were read visually. This type of system requires that the drop of blood is deposited at the right moment to start the analysis, that the reaction time is controlled until the blood is eliminated from the strip, and that this latter operation is also carried out manually, by drying or wiping. These three steps are potential sources of variability in the test results. The One Touch system was designed to exploit all the advantages of a reactive strip that is read instrumentally and to eliminate the intervention of the user in these three steps, thus eliminating the sources of variability. This advance in design over previous systems makes One Touch a "second generation" system.

There are three technical elements of the One Touch system that distinguish it from the first generation systems:

    1 - The specific porous hydrophilic membrane of the reactive strip.
    2 - The opto-electronic system that detects the application of a sample to the reactive strip.
    3 - The bichromatic optics system that accurately reads the colorimetric reaction, even in the presence of erythrocytes.

Each of these three key elements has specific functions that reduce the variability of the results of the analysis.

The One Touch reactive strip incorporates the already established chemistry of glucose oxidase/peroxidase in a specific microporous hydrophilic membrane with a structure similar to a microscopic sponge. The membrane has three purposes: 1) it acts as a reservoir to store an exact amount of sample, 2) it acts as a filter to exclude the discrete elements of the sample and 3) it acts as a smooth optical surface for making reflectance measurements.

This third characteristic is essential for the measuring device to read the lower surface of the strip while the blood remains on the upper surface. Moreover, the membrane's hydrophilic properties facilitate the application of blood upon the analysis area.

Once the sample is applied to the reactive strip and penetrates the membrane, the measuring device detects a sudden change in reflectance and starts the 45-second time sequence. Thus it is the measuring device, instead of the user, that controls the start of the analysis.

The time control until the blood is eliminated or cleaned from the strip and the elimination itself, whether by means of drying or wiping, are eliminated in the One Touch system by a system of bichromatic optics. The best way of describing this technology is by explaining the analysis process in detail.

To carry out a glycemia measurement with One Touch, a reactive strip is inserted in the One Touch measuring device and the latter is switched on. The measuring device takes a zero reading from the unreacted strip and instructs the user to "APPLY THE SAMPLE". When the whole blood sample is placed on the surface of the membrane the plasma is rapidly absorbed towards the membrane while the erythrocytes and the excess plasma are excluded and remain on the surface. When the sample penetrates the membrane, the measuring device detects a change in reflectance and starts a 45-second sequence. The blood glucose reacts with the oxygen catalysed by the glucose oxidase (GO) and forms hydrogen peroxide ($H_2O_2$), which subsequently reacts with the chromogens of the indicator with the aid of horseradish peroxidase (HPO) in accordance with the following equation:

$$\text{Glucose} + O_2 \rightarrow\ ^{GO} \rightarrow \text{Gluconic Acid} + H_2O_2$$

Within 45 seconds the reaction is over and a reflectance reading is taken. The indamine chromogen, formed as a result of the chemical reactions described in the aforementioned equation, absorbs the light from a first light-emitting diode. However, the components of the blood in the reaction mixture and the excess from the sample also absorb at the same wavelength of this diode.

In order to correct this background interference, a second sample is taken with a second light-emitting diode, but with a different wavelength peak that detects the blood but not the chromogen. When these two signals are substracted, the interference signal is isolated. The chromogen signal thus generated is correlated with the glucose concentration by a calibration process. This is done by measuring the glucose concentration in whole blood with the glucose analyzer (Yellow Springs Instrument Model 23A), which uses glucose oxidase chemistry with electrochemical detection of peroxide. The optical signal for a batch of One Touch reactive strips is related to the glucose concentration by means of a calibration curve. The calibration codes are a family of mathematically related curves that adjust to the differences in response of the batches of One Touch reagents. Using this method, the One Touch system measures the glucose concentration in whole blood.

THE BACKGROUND OF THE AMPEROMETRIC BIOSENSOR

This type of biosensor is based on the electrochemical method of analysis by amperometry. It is a method that uses a very small amount of electricity which does not alter the composition of the sample. The reactive strip is made up, in this case, of three electrodes: the working, auxiliary and reference ones.

In this technique the potential between the working and auxiliary electrodes is fixed and the intensity of the current generated during the reaction is measured. During this reaction the potential between the working and the auxiliary electrodes may vary due to changes in the concentration of polarization and overpower (phenomena which are characteristic of electrolysis), for which reason a third electrode is used, the reference electrode, in order to achieve the fixing of potential. The materials used in the electrodes must be antioxidants (graphite, silver or silver chloride are generally used).

The current intensity is a function of the sample concentration, and also of the potential. In certain conditions, it is possible to deduce, after a previous calibration, the concentration of the electrolyzed substance by measuring its intensity. The intensity measurement allows the variation in concentration to be followed through the course of the chemical or electrochemical reaction, by means of which the titration reaction is controlled (the titration consists of following the current strength throughout a chemical reaction).

The principle on which the amperometry is based is the polarography (a term used initially in electrolysis reactions in which a working electrode was used, called "mercury drop"). In polarographic determination, electrolysis is carried out with a reference electrode; by varying the voltage of electrolysis, the curve $i = f(E)$ is traced. For calibrating several curves are made at different glucose concentrations. Then the height of the diffusion wave is measured, which is proportional to the concentration of oxidised glucose in the working electrode: $i_d = K_d \cdot c$, where $c$ is the concentration (known during calibration) of the glucose.

For the oxidation reaction to reach its saturation point (the moment at which the intensity reaches maximum value) it is necessary to apply a minimum potential in accordance with the reaction:

$$Re \rightarrow Ox + e.$$

The value of the constant Kd depends on the dimensions and the geometric arrangement.

The basic electrical schedule is based on a voltage source of constant potential V (normally of 650 mV). For the intensity reading at the galvanometer G to be valid the difference in potential between two points, A and B, must be constant (we know that it must be $i_1$ x $R_1$, and when the intensity $i_1$ is saturated, the potential is constant). This schedule is valid once it is calibrated. The calibration in this case would consist in obtaining a suitable value for the resistance $R_1$ so that the potential between points A and B is sufficient to reach the saturation current when the concentration is at the maximum. With this difference in potential there will be possible to measure the saturation current for any concentration lower than the maximum obtained. The calibration process would be carried out by measuring the current intensity of different known concentrations, and calculating the parameters of the linear regression line associated with the concentration-intensity pairs. Once calibrated, we may approximate by interpolation the concentration of the sample to be analysed. The calibration is valid for reactive strips which have an identical reactive composition, or with very small variation margins. Otherwise, it will be necessary to re-calibrate the instrument.

The chemical reaction that takes place in the cell is the following:

$$GO \ (Glucose) + O \rightarrow Gluconic \ acid + H_2O_2 \quad [1]$$

$$H_2O_2 \rightarrow O_2 + 2H + 2e \quad [2]$$

In reaction [1], the glucose, together with the oxygen, reacts in the presence of the Glucose Oxidase (GO) enzyme to generate gluconic acid and hydrogen peroxide ($H_2O_2$). The trigger of the reaction [2] is the potential V. The electrons that are released give the measure of the current, which is proportional to the glucose concentration.

It should be mentioned that the reactive strip on which both the reagents and the potential-measuring electrodes are found, is made up of a PCB (Printed Circuit Board), which although it has a significant physical consistency, involves an additional cost.

Like most diseases, diabetes has two vulnerable points: timely diagnosis and suitable treatment. But, unlike other diseases, the serious individual and social consequences of diabetes may be substantially reduced by something that is as simple in theory as it is complicated in practice: the precise and periodic control of blood glucose levels.

The process is simple in theory because the pharmaceutical laboratories have attained extraordinary levels of accuracy in the measurement of blood glucose. But it is complicated in practice because, to be effective, the result of the measurement of blood glucose has to overcome three obstacles: interpretation, time and distance.

Rigorous control of glucose levels reduces the risks of diabetes, but triples the danger of suffering decompensation due to hypoglycemias. That is, the figures have to be interpreted in their context and by personnel qualified to do so.

Secondly, the interpretation must be carried out at the right time, as close as possible to when the measurement was taken, which is when decisions have the greatest importance. And, finally, the geographical distance between medical staff and the patient should not be an impediment for interpretation and decision making.

There are patents that attempt to resolve this dual problem of self-analysis and data transmission in real time. Thus, patent WO94/12950 presents us with a glucose analysis device, with the possibility of printing the results obtained and storing the records of several patients. However, it does not feature processing of these data nor the possibility of telematic interaction with a central data processing unit. Patents WO94/11831 and EP483595 partly resolve the problem by allowing interaction between a microprocessor connected to the glucose analyzer and a remote computer that receives and supplies data through the respective interfaces. The limitation of this apparatus is that it requires communications software via modem to send data to the processing unit. The latter in turn sends a report concerning the data received to the peripheral units via fax. We can see therefore that different apparatus dispersed in different locations are necessary to send the data, and so transmission in real time begins to lose its advantages. In other cases, as shown in patents WO94/06088 and EP462466, monitoring of the patient is automatic. The medication programme includes an entire series of parameters adapted to different situations that generate automatic responses. However, this type of system and the apparatus that implement them lacks the added value of the medical expert's judgement at all times, as well as having little adaptability to unforeseen emergency situations.

These obstacles, which until recently were economically and technically unsolvable, can be overcome today thanks to the vertiginous advance of new technologies. The integration into a single apparatus of the glucose measuring device together with a small computer and a mobile telephone allows the creation of a solid system of management and control for as many patients as the respective systems wishes to include. At the same time as patients carry out their glucose measurements, the computer stores the resulting figure and sends it to a central computer that stores and controls the measurements of all the patients assigned to it. The present coverage of mobile telephone systems ensures that on-line transmission of the figure is practically totally guaranteed.

**Summary of the Invention**

The central unit, integrated into a hospital service or reference centre, would be connected to the patients' units, on the one hand, and to the peripheral units of doctors, laboratories, patients, etc., on the other.

The central computer has sufficient information about the medical history of each patient to decide in which circumstances it should alert the medical service when the figures exceed what is permitted for that patient. The patient may also use the mobile telephone to make immediate contact with his doctor or with the Diabetological Unit that attends him, and the corresponding medical service can send the appropriate instructions at the same time.

This is of particular importance in rural areas, with diabetic patients scattered over a wide geographical area, who are cared for by a family doctor with responsibility for that area. In the event that the patient or the central computer considered the situation to be alarming, contact between doctor, patient and the Diabetological Unit would be immediate and direct, so that the most appropriate decision could be taken without the need for waiting or long journeys.

This system allows coverage in the case of emergencies or staff holidays to be perfectly fulfilled, so that the specialist may temporarily assign control of his patients to another doctor or to the Central Unit's own emergency service.

In principle, the system has been designed for the control of blood glucose levels of diabetic patients, but by the same procedures and with the same technical set-up, the control of all physiological variables that may be transmitted telematically could be incorporated.

Each doctor, whether this be a specialist in a city or the family doctor in a small village, establishes the range of what is to be considered alarming for each of his patients. If a patient in a remote village is suddenly taken ill, or if their routine analysis indicates abnormal figures, the central computer will instantly communicate this circumstance to the doctor responsible for that patient, who will give instructions by telephone to the patient regarding what is most appropriate in his situation.

The system is especially indicated for patients who have been recently diagnosed; patients who suffer from other complications; pregnant women; elderly patients; young children; people who live alone or in remote geographical areas; patients who engage in dangerous activities or who travel frequently, etc. To summarize, the developed invention allows hospitalization to be replaced in many cases. Moreover, there is the possibility of multiple or collective use. It is clear that a device with these characteristics could be very economical for groups such as residential homes, barracks, schools, work places, etc., where a single unit permanently controls any emergency situation for any members that might need it. In the same way, the monitoring of patient with the invention may be temporary, according to the specific situations that might justify it.

The present invention provides a series of advantages over the known state of the art, which may be enumerated as follows:

.    By means of the advanced technology used, it reduces the patient's dependence on traditional medical services, thus improving their quality of life.
.    Thanks to constant and personalized monitoring, it prevents critical situations from arising in patients which might pose a serious health risk for them, and sometimes even cause death.
.    It prevents expensive hospital admissions and appreciably reduces patients' work absenteeism, thus palliating the social and personal impact caused by the disease.
.    It facilitates treatment control and application by patients, thus increasing the health care capacity of the medical services.

When measuring blood glucose, the patient receives not only a figure, but also the interpretation of that figure according to his specific circumstances. In the unit's viewer the patient may confirm that his situation is: "Alarm", "High", "Low", "Acceptable", depending on his evolution and the judgement of the specialist treating him. Secondly, the patient knows that, wherever he is, at the other end of the line there is the Diabetological Unit or Hospital Centre, as well as the specialist who treats him, or his own family doctor, as the case may be.

**Detailed description of the invention**

The system is made up of four basic elements or subsystems that communicate with each other in real time. These elements are:

■    CENTRAL UNIT.- A subsystem in which the communications of patients' Mobile Units and the doctors' Units are centralized. The Central Unit has an Indexed Data Base List that includes samples and information generated by the Peripheral Units, for example, the patients' Mobile Terminals and those of the doctors, and makes them available to these units in accordance with the system's requirements. This subsystem has the following components:

1.1.1 Interconnected fault tolerant server computers
1.1.2 Multi-area operating system
1.1.3 Consultation terminals
1.1.4 Local area network
1.1.5 Data base
1.1.6 Management, monitoring and control programmes
1.1.7 Router communications and links with X.25 port or similar and configurable to different transmission speeds.
1.1.8 Point-to-point communications and links at different transmission speeds, with different communication protocols and virtual switched circuits, connectable to different types of modems (including those providing access to digital communications via radio, such as GSM, DCS, PCN, etc.).
1.1.9 Complete electrical energy supply system, with uninterruptable supply unit, connections and protections.
1.1.10 Communications components and links, with patients' terminals, doctors' terminals and laboratory terminals and whatever other terminals are necessary for the exchange of information and data.
1.1.11 Auxiliary components: printers, screens, etc., needed for normal and complete operation of the Central Unit.

■    LABORATORY TERMINAL.- A subsystem with a fixed terminal (PC) or a mobile (portable) one, that can introduce analytical data relating to a particular patient into the System by means of communication with the data base of the Central Unit. This subsystem has the following components:

1.2.1 Personal computers (PCs)
1.2.2 Operating programmes for the personal computers
1.2.3 Data bases for the personal computers
1.2.4 Modems for connection to fixed or cellular telephone networks, whether analog or digital.
1.2.5 Fixed or portable cellular telephones, whether digital or analog.
1.2.6 Other components for interconnection with the Central Unit, patients' terminals and doctors' terminals.

■ DOCTOR'S UNIT. - A subsystem with a doctor's portable terminal, fixed (PC) or portable, which attends to the patients' Mobile Units by means of communication with the Central Unit, from which it receives the information deposited in the said centre by the patients' Mobile Units, and to which it sends the specific information for the monitoring and control of the patient. This subsystem has the following components:

1.3.1 Personal (PC) or portable computers

1.3.2 Operating programmes for the computers

1.3.3 Data bases for the computers

1.3.4 Modems for connection to fixed or cellular telephone networks, whether analog or digital (GSM, DCS, PCN, etc.)

1.3.5 Fixed or portable cellular telephones, whether digital or analog.

1.3.6 Specific programmes for control and monitoring of patients' medical parameters, and for monitoring and dialogue with the Central Unit, laboratory terminals and between medical terminals.

1.3.7 Communications, linking, control and monitoring programmes and apparatus of the Central Unit, patient terminals, laboratory terminals and other doctors' terminals.

With respect to communications between these elements, whilst the patient's mobile terminal system uses the Short Message Service (SMS) of GSM as a transmission channel, the medical and laboratory terminals will connect via GSM (9,600 Bps).

The explanation for this choice is as follows: the mobile unit generates and receives very low information traffic, which may be carried efficiently in terms of cost, speed and security by the Short Message Service (SMS), which is based on the transmission of packets of 160 character signals in the GSM network. However, the doctor needs access to a great volume of information about each of the patients he attends, to do this he requires greater speed to carry out this task in a short and convenient response time.

- PATIENT'S TERMINAL. - A mobile subsystem with a patient's terminal, based on the combination of a computer a GSM mobile terminal and a Blood Glucose Level Analyzer, in which glucose samples are collected and the necessary data are introduced to enable the doctor to control and monitor the patient. It receives from the Central Unit the specific information generated by the doctors to attend to each patient. This subsystem has the following components:

1.4.1 Patient's personal computer of small size (of the type called personal notebook, palm-top, pocket calculator, ADP, etc.), including both commercial and specific hardware and software.

1.4.2 Patient's mobile, digital cellular telephone terminal (GSM, DCS, PCN, etc.), with its programmes and utilities.

1.4.3 Glucose-measuring autoanalyzer.

1.4.4 Controller (CPU) whose function is to control the patient's terminal. Its functions range from dialling the telephone number of the other subsystems to capturing data from the glucose measuring device in order to subsequently store it in the patient's personal computer and send it to the other terminals or to the central unit. This in turn has the following components:

1.4.4.1 Printed circuit board.

1.4.4.2 Low-consumption CMOS microprocessor.

1.4.4.3 Non-collateral RAM memory for data storage.

1.4.4.4 Flash EPROM memory for storing the application programme.

1.4.4.5 DES cryptography module to ensure communications.

1.4.4.6 SMS digital interface with GSM terminal (or with DCS, PCN, etc.).

1.4.5. Battery to provide power.

1.4.6. Moulded body or housing in which the personal computer, telephone and glucose analyzer are integrated.

1.4.7. Operating programmes for management and communication of the different parts of the patient's terminal with each other and with the terminals or the central unit.

From the functional point of view, the proposed unit must fulfill a series of functions all of which tend to provide medical services with the correct application of treatment for their patients in the shortest time possible, above all in emergency situations. These functions are detailed below.

. Analysis of the amount of glucose in the patient's blood sample, and transmission of these data to the patient's terminal.

. Communication of blood glucose values received by the patient's terminal (from where this is situated) to the central unit.

. Maintenance of a data base (resident in the patient's apparatus) with all the values of the analyses carried out.

. Display and statistical evaluation of the medical record data resident in the patient's computer.

. Evaluation of possible critical situations, generating the corresponding alarms and/or messages to the personnel responsible for monitoring the patients.

- Display of patients' medical records, stored in the central unit, which help the doctor to reach conclusions, and consequently, to apply the appropriate treatment.

- Statistical evaluations according to different criteria of patients' data stored in the central computer.

**Example of a preferred embodiment of the invention**

A preferred embodiment of the practical application of the invention is described hereunder, broken down into each of its different components. Variations that do not alter the operation or the basic operating principles of the invention are to be understood as covered by the scope of protection that may eventually be granted to the same.

PATIENT'S TERMINAL

The patient's terminal is made up of a blood glucose autoanalyzer, a compatible personal computer and a digital mobile telephone apparatus (GSM), integrated in a single apparatus.

The technical features of the apparatus in its entirety are indicated below.

1.4.1 Patient's personal computer of small size (of the type called personal notebook, palm-top, pocket calculator, ADP, etc.), including both commercial and specific hardware and software.

1.4.2 Patient's mobile, digital cellular telephone terminal (GSM, DCS, PCN, etc.), with its programmes and utilities.

1.4.3 Glucose - measuring autoanalyzer.

1.4.4 Controller (CPU) whose function is to control the patient's terminal. Its functions range from dialling the telephone number of the other subsystems to capturing data from the glucose measuring device in order to store it subsequently in the patient's personal computer and send it to the other terminals or to the central unit. This in turn has the following components:

1.4.4.1 Printed circuit board.

1.4.4.2 Low-consumption CMOS microprocessor.

1.4.4.3 Non-collateral RAM memory for data storage.

1.4.4.4 Flash EPROM memory for storing the application programme.

1.4.4.5 DES cryptography module to ensure communications.

1.4.4.6 SMS digital interface with GSM terminal (or with DCS, PCN, etc.).

1.4.5 Battery to provide power.

1.4.6. Moulded body or housing in which the personal computer, telephone and glucose analyzer are integrated.

1.4.7. Operating programmes for management and communication of the different parts of the patient's terminal with each other and with the terminals or the central unit.

CENTRAL UNIT

This element has the characteristics belonging to a centralized computer system made up of a Data Server, a Sentinel Computer, a Communications Router, one or more portable computers for the use of the doctors responsible for monitoring the patients, a Printer, an Uninterruptable Supply System and a Back-up System for backup copies of the information.

The computer called Sentinel has the task of constantly evaluating the results received from patients, as well as sending alarm messages when appropriate. In this way, the available human resources may be directed to decision making or patient care.

The apparatus called the Router has the task of channelling the communications between the patients and the central computer, making possible simultaneous multiple connections. The use of this type of apparatus in the communications environment enables remote users to access software in a totally transparent way. This avoids the use of special protocols which unnecessarily lengthen the process of using the software.

The technical characteristics of each of the components of the centralized system are indicated below:

Data Server

- 100 Mhz Intel Pentium Microprocessor
- 16 Mb RAM memory
- 1 Gb hard disk (SCSI)
- 3 1/2" diskette unit
- Colour monitor
- High-performance 32-bit network card (PCI)
- High-performance 32-bit disk controllers
- Novell network operating system for 50 users
- Advantage Xbase server client module for 10 users

Sentinel Computer

- 66 Mhz 80486 Intel microprocessor
- 4 Mb RAM memory
- 540 Mb disk
- VGA colour monitor
- 16-bit network connection card

Communications Router

. Compatible with IPX protocols
. Ethernet 802.3 compatible
. Capacity for running 4 simultaneous connections (ideally 16)
. Capacity for cascade connection with other units of the same type
. Capacity for managing user access by means of passwords

Doctor's Set (Notebook)

. 50 Mhz 80486 Intel microprocessor or better
. 4 Mb RAM memory
. 540 Mb hard disk
. 3 1/2" diskette unit
. Colour monitor
. Network card

Printer

. Laser quality (preferably from the Hewlett Packard 4 series)
. Printing speed of between 8 and 12 ppm

Uninterruptable Supply System (SAI)

. Approximately 5,000 Watts of power
. Autonomy time from 20 to 30 minutes
. Capacity for automatically activating the network shut-down mechanism

Back-up System

. 4 mm DAT tape system, with SCSI interface, connected to a high-performance network work station (this means that the said station must have an SCSI controller installed).

The Central Unit will interact with a whole series of peripheral units, receiving and sending information from and to each of them. For this purpose its structure will have a series of modules specifically designed to communicate with each of the said peripheral units. These modules, their function and components are described in detail below.

CONFIGURATION OF THE CENTRAL UNIT

The Central Unit features a Local Area Network with two computers: a dedicated fault tolerant server, loaded with a Windows NT multi-area and multi-user operating system (SERVER option) and a consultation Terminal, also loaded with Windows NT (WORKSTATION option).

For the analysis, collection and management of samples from the Central Unit, the Microsoft Access Indexed Data Base will be used, on which the application will be developed from which both the SYSTEMS and the MEDICAL TERMINALS will gain access.

So that all communications may be carried out by digital or analogue, cellular mobile telephone, the Central Unit is to have the following elements:

- A Router with a X.25 port configurable to speeds of between 64 Kbps and 2 Mbps.
- A 64 Kbps point-to-point link between the Central Unit and the telephonic communications manager. This link will support the X.25 communications protocol and, in principle, a minimum of 10 (ten) Commutating Virtual Circuits (CVC's), which initially will allow up to a maximum of 10 doctors with a GSM modem to operate simultaneously. This link may be increased in speed and number of CVC's to allow simultaneous access to a greater number of doctors.

To ensure the system's operation in the face of any failure of apparatus or power, Fault Tolerant redundant computers must be used with a double CPU, Source, Hard Disk, etc. configuration, and an Uninterruptable Power Supply (UPS) that guarantees a power supply to the Central Unit when there is a power failure.

The Central Unit will also be provided with a laser printer connected to the network for sending documents.

The Central Unit will be responsible for registration and cancellation of doctors' terminals and laboratory terminals in the System.

If a terminal is not registered, they can not be connected to the central unit for receiving or sending information.

When a patient operates with the System, he will be able to check that the apparatus is registered by seeing whether his personal details appear on the screen. At the initial registration, in addition to his personal details and those of the doctor who attends him, the System receives basic information about operation, and information relating to medication, diet, controls, clinical reports, etc. This information is generated by the doctor and it is an indispensable requirement for it to be introduced into the Central Unit's data base so that the patient may use the system.

Doctors will have access to monitor the information about their patients and the information that they generate, if they enter the correct code when they link up with the Central Unit.

Laboratories will have access to enter analytical data concerning a patient into the Central Unit's data base, if they enter the correct code when they link up with the Central Unit.

The process of cancelling a medical terminal or a laboratory terminal is carried out by deleting the password the System requires from them when they connect. This process does not mean that the data introduced into the system by the doctor, the laboratory or the patient will be lost.

In any case, all the information generated in the System will remain registered and stored in the Central Unit's data base for future analysis.

PATIENT'S MODULE

Its fundamental function is to make the data stream automatic between the patient and the doctor. It is made up of the following components:

* Autoanalyzer-computer communication in the patient's terminal.

This component sends the glucose values from the autoanalyzer to the computer of the patient's terminal each time an analysis is carried out. The programme receives a numeric value from the autoanalyzer that is converted into a screen message (Alarm, High, Low, Acceptable) according to the reference values preestablished for the patient in each period of the day.

The values may be modified by the personnel responsible for controlling and monitoring patients (mainly by the doctor) as will be seen in the Patients' Master Maintenance programme.

* Patient-Central Unit terminal communication

This component will send all the values resulting from the measurements taken by patients (glucose, weight, blood pressure, height and type of physical exercise performed) to the Central Unit. In principle this communication will take place every time the patient's set receives a value from the autoanalyzer. However, in the event of any unforeseen event (communications failure, failure of power supply to the apparatus, etc.) the communication will be re-tried as many times as necessary.

As this communication is controlled by computer mechanisms, the data sent to the central unit will be more reliable and regular in time. As already mentioned, this aspect is of fundamental importance for the application of adequate treatment to the patient.

Moreover, the patient may also generate requests for help from his set. This will be possible as long as the said service has been set up for that patient by the personnel responsible for the centralized system.

* Data base maintenance

All the biochemical analyses (glycosylated haemoglobin, fructosamine, cholesterol, LDL, HDL and triglycerides) and controls (glucose, weight, blood pressure, height and type of physical exercise) carried out and sent to the central computer will also be stored in the patient's set. This will facilitate the visual display of the patient's medical records by other medical services according to the same criterion observed in the central computer.

* Communication between the central unit and the patient's terminal

The doctor will be able to send to the patient changes in his system parameters (eg. doses of insulin to be injected, frequency of glucose controls, etc.) which will be stored in the data base of the patient's set; or indications concerning his treatment in the form of electronic mail messages. These messages will remain in the patient's set until he has read them and voluntarily deleted them.

From the technical point of view, the data that must be sent to the patient's set will be deposited by the Sentinel computer in a part of the Central Unit's disk that corresponds to the patient (called mailbox) and collected by the patient each time he connects in order to send data concerning the controls carried out. Likewise, the memory of the patient's set could, when necessary, be available to other specialists.

* Visual display of the patient's medical records

The data stored in the patient's set may be viewed by the same or by any medical service. In this way, the patient's records may be made available whenever necessary. The said records may be viewed in accordance with the following criteria:

Evolution of the Glucemic Profile. All the data relating to glucose measurements taken in a specific period of time may be viewed on screen, in the form of text. The values corresponding to the periods in which the patient has done physical exercise will be seen highlighted by a different background so as to be able to easily appreciate its effect on the glucaemic profile. Likewise, values that are beyond the range will appear highlighted in a different colour.

The detailed study of these data by the doctor will enable the tendencies arising in the patient's glucemic profile to be determined and, consequently, the reasons for each variation, thus facilitating the application of adequate treatment. The evolution of the Glucemic Profile may be viewed as text (in table form) or in graph form.

As this information is displayed in graph form, it provides the doctor with a reading of the tendencies of the glucaemic profile according to hours, days, weeks, before and after meals, etc..

In addition to the evolution of the glucemic profile, the visual display programme allows the following data to be seen on screen:

Weight evolution
Height evolution (Children)
Blood pressure evolution
Glycosylated haemoglobin evolution
Fructosamine evolution
Cholesterol evolution
LDL evolution
HDL evolution
Triglyceride evolution

THE SENTINEL MODULE

This module releases those responsible for running the centralized system from the routine and constant task of receiving data and evaluating critical situations that correspond to reference patterns. It is made up of the following components:

* Reception of data sent by the patient

This component will be in permanent execution in the computer called Sentinel and its task will be to carry out a constant scanning of the communications mailbox of the data server so as to incorporate any data received into the records of the corresponding patient. As soon as this occurs, the data received will be available to the other programmes of the application.

* Assessment of critical situations

Once the data are incorporated into the patient's records, they will be compared with the preestablished values for that patient, for that period of the day. This comparison will enable the Sentinel computer to determine whether the patient's glucose values are within normal values or not.

* Transmission of alarms and messages

The purpose of this component of the system is to transmit alarms and messages to the doctor responsible for monitoring each patient when the Sentinel computer has assessed a situation as critical, or when the patient has sent a request for immediate care. For each new message the doctor's computer will emit an audible signal, and will indicate how many messages are waiting to be read by means of a message on screen (in a different colour). If any of these messages recommends verbal communication between doctor and patient, this will be done by telephone.

Messages are to be marked with the time at which they were read by their addressee. In this way the doctor responsible may establish with total accuracy the response time given to patients.

* Daily monitoring of patients

As each patient sends data from his controls to the central unit, the Sentinel apparatus will display in a line on the screen the patient's identification and data relating to the control carried out. Values above or below the reference values will be seen in a different colour; and those corresponding to the control times preestablished by the doctor will be seen on a different background.

This tool will help the doctor to determine in a simple way whether patients carry out their controls at the established times, and deviations from a patient's reference values.

At the user's request, all patients can be seen grouped according to the health centre to which they belong, instead of seeing them in chronological order according to the time of transmission.

MEDICAL MODULE

This is the instrument that will allow the doctor to perform the tasks of control, monitoring and statistical assessment of each patient in a personalized way, thus facilitating the application of treatment for each case. The module is made up of the following components:

* Maintenance of the patients' master records

By means of this component of the system, the personnel responsible for the central unit (or else the doctor responsible for the patient) will carry out the maintenance (registration, cancellation, modifications and consultations) of the patients' master record. Each patient is to be identified by a single random number generated by the system, in order to avoid duplication or loss of data.

In addition to personal details (name, age, doctor responsible for monitoring, etc.), the programme will allow the patient's levels or ranges of glucose to be updated for each period of the day.

* Maintenance of the doctors' master records

By means of this component of the system, the personnel responsible for the central unit will carry out the maintenance (registration, cancellation, modifications and consultations) of the master record of doctors associated with the system.

* Message reception

The messages sent by the Sentinel advising doctors of critical situations or requests for help from their patients will be managed by this programme. The doctor will see a warning in a different colour on his screen indicating the number of messages waiting to be read, followed by an audible signal if the message that has just arrived concerns an emergency situation.

* Medical records

Each patient's records are to include their Medical Records. For this purpose, it will be possible for the doctor to include information from each section of the medical records as it arises. There will also be the possibility that the central unit may communicate directly with patients themselves, in case of emergency or absence of the doctor responsible.

The different sections of the medical records are indicated below.

- Reason for the consultation
- Current disease
- Personal background
- Family background
- Haematometry
- Diabetological profile
- Lipidic study
- Renal study
- Biochemistry
- Fondus oculi
- Cardiovascular assessment
- Electro-cardiogram
- Other tests
- Systematic manifestations
- Clinical opinion
- Treatment
- Evolution

As time passes and the disease evolves in one direction or another, new data is produced that must be included in the above-mentioned sections. Likewise, part of the old information may undergo alteration due to new occurrences in the patient's life.

By using this programme, the doctor will have at his disposal the tools that will allow him to keep the medical records up to date, whether by including information as it is produced, or by modifying old data or changing them from one section to another as the case may be.

* Control and monitoring

This programme is an instrument of fundamental importance for helping the doctor to control and monitor his patients. Its use allows him to determine:

- Whether patients carry out their analyses at the established times.
- Deviations from the established reference values.
- The effect of physical exercise on the patient's evolution.
- Etc.

* Statistical assessment

This system's record visual display functions (as described in the section "Patient's Module") are made up of a series of programmes which after data statistical assessment, display the information on screen according to different criteria. These functions will allow the doctor to assess the effect of multiple factors on the patient's evolution in perspective.

CONFIGURATION OF THE SYSTEMS

According to the application of the systems, three types of operation are distinguished:

- PERSONAL: The set is the property of a single patient who uses it exclusively and it will always be personalized with his data.
- HOSPITAL: The set belongs to a hospital department and is used by its personnel to take the measurements of a limited number of patients. According to the capacity of the sets, these will record the information of a certain number of patients and by means of function keys with which the set is provided, measurements may be alternated from one patient to another.
- HEALTH CENTRES: The set is assigned to a Health Centre which hands it over to a patient for a period of time to monitor his disease. Each time the set is handed over, the Central Unit must register the patient.

The application of the system will be prepared to adapt to any of the previous modalities. In the first and third cases, only one patient will operate the set. In the second case, the maximum number of patients to which the set may be applied will depend on the ultimate memory capacity.

CONFIGURATION OF THE MEDICAL AND LABORATORY TERMINALS

The medical and laboratory terminals do not require a special configuration to operate in the system.

Any apparatus loaded with WINDOWS NT in its USER option, with the Microsoft ACCESS data base, the application programme, a GSM modem and a mobile terminal with data transmission capacity, may access the system and operate without difficulty.

PARAMETERS TO BE MONITORED

Both the patient's terminal and the medical terminal can be accessed to monitor the following parameters:

- Evolution of blood glucose level
- Weight evolution

- Height evolution
- Blood pressure evolution
- Evolution of the following parameters of blood analysis:

  1 - Cholesterol
  2 - HDL-C
  3 - LDL-C
  4 - Triglycerides
  5 - Fructosamine
  6 - Haemoglobin Alc.

These parameters must be shown but never modified, neither in the patient's mobile terminal, nor in the doctor's terminal, as they are vital for giving a diagnosis on a determinate patient.

In all cases, it is the patient's and the doctor's responsibility to enter the data correctly into the system's mobile terminal. In order to prevent false information, the mobile terminal set will be provided with sufficient control and validation mechanisms before such information is definitively entered into the Central Unit's data base. Any erroneous information will be corrected by introducing subsequent information that will prevail over the former information.

To enter the above-mentioned parameters, except for the blood glucose level which is carried out automatically without the intervention of an operator, there are different dialogue screens (weight, height, blood pressure, etc.).

The data may be entered manually by the patient, the doctor or the laboratory that carries out the corresponding analysis. In the patient's case, he will do this on his mobile terminal. In the doctor's or laboratory's case, they will do it by means of a direct connection with the Central Unit. In any case, all data introduced into the system will remain stored in the Central Unit's data base to ensure the perfect subsequent monitoring of the flow of information.

With respect to blood glucose levels there are a series of programmed daily measurements, which the doctor will prescribe to the patient from his terminal: before breakfast, after breakfast, before lunch, after lunch, etc.. In addition to these, the patient may carry out as many as he wishes, although the set will only display up to a maximum of 24 daily samples. However, the doctor will be able to gain access from his application in order to find out the value of all the measurements carried out.

STATISTICS

The values recorded in the mobile terminal will serve to generate statistical information of medical interest for the patient.

The statistical values are to have two forms of representation: tables and graphs.

When the table form of presentation is chosen, the patient will have access to all the recorded data, which will be presented in a list by means of the scrolling function, so as to overcome the limitation of the screen's size. The graph form will graphically represent the measurements of the said portion of the table.

The evolution of the blood glucose level will be accompanied by the following measurements:

- Average value of the programmed hourly samples
- Complete average value of all the daily samples
- Standard deviation from the average
- Exercise performed
- Change of dosage

The other graphs represent the evolution of other measurements (weight, height, blood pressure, etc.).

All the graphs will be two-dimensional in linear format.

This same information, with even more detail, will be accessible from the doctor's terminal.

TREATMENT

The system will provide the patient with the following information about the treatment prescribed for him by the doctor:

- MEDICATION: indicates the medicines and dosage he must take.
- CONTROLS: indicates the controls and when they must be carried out.
- DIET: indicates what diet he must follow.

This information is generated by the doctor from his terminal, introduced into the data base and sent to the patient via GSM.

ALARMS

The doctor will generate a table with the blood glucose levels, which indicate to the patient the state of metabolic control associated with each analysis carried out at each period of the day.

CLINICAL REPORT

As in the case of medication, the system stores the patient's up-dated Clinical Report. This information is generated by the doctor from his terminal, introduced into the data base and sent to the patient via GSM. At the patient's request or at the request of any other doctor who attends him, it may be displayed on the screen, although it cannot be modified.

FUNCTIONAL DESCRIPTION OF THE PATIENT'S TERMINAL

The operation of the patient's mobile terminal is described below.

ICONS

There are eight function keys in the TERMINAL, which give access to the eight general basic functions of the set. Pressing each of them gains access to the use of the different options associated with them.

These eight keys and their associated options are represented in a structured way below.

1. - TREATMENT

Shows the treatment prescribed for the patient by the doctor.

This information is read-only and cannot be altered by the patient. That is, the doctor edits the information and enters it into the system; subsequently he sends it to the patient via GSM and it is stored in the set until a new Treatment is received.

2.- ANALYSIS

Allows a glucose level analysis to be made and displays the data obtained.

When the patient selects this function the system activates the internal blood glucose level analyzer.

As in the case of analyzers with conventional reactive strips, the set indicates the steps that the patient must follow to complete the analysis successfully.

The analyzer's measurement is automatically recorded in the memory of the set and sent to the Central Unit's data base. The doctor may gain access to this information and monitor the patient's evolution when he connects and requests his data.

The mobile terminal is capable of displaying up to 24 blood glucose level measurements a day (one per hour). When a measurement is taken, it is associated with the time it was taken.

Information concerning the measurement value and the hour and minute it was taken is sent to the Central Unit.

In the event that the patient takes more than one measurement per hour, the set will advise the patient of this fact, but will represent only the last measurement taken in the said interval. However, when the table of the 24 daily measurements is displayed, the fact that there is more than one measurement in an hour will be indicated. When the doctor detects an anomaly in one of the table's values, he will have the option of inspecting each measurement taken in the said period.

When data are entered incorrectly, the patient may indicate this fact by sending a message to the Central Unit.

Together with the measurement, a table of values generated by the doctor is displayed, indicating the state of metabolic control associated with the same, according to the time of day it is taken. If the maximum or minimum alarm thresholds are exceeded, this information will be given to the patient so that he may call his doctor.

3.- ENTERING DATA

By means of this function the patient may enter data into the mobile terminal's computer. These data are very varied and constitute a relevant complement of the blood glucose level measurements.

When the patient selects this function, the system displays six options on screen:

- I EXERCISE: This helps the patient to enter the periods of exercise he performs during the day, such as sport, gymnastics, etc.

  This information is important for the doctor to be able to correctly interpret variations in blood glucose levels.

- II SELF-DOSAGE: When the patient changes the dosage prescribed by the doctor, he may use this function to record the new dose he is taking.

  As in the case of exercise, this information is of great importance for the doctor.

- III BLOOD PRESSURE: The patient may store in the set the blood pressure measurements (maximum and minimum) he carries out. The set will record the values of the measurements and the date and time they are carried out.

- IV HEIGHT: This item is important in the diagnosis of children in growing age. It is a measurement spaced out over time. The set will record the value of the measurement in centimetres and the date and time it is carried out.

- V WEIGHT: The patient's weight measurements may be recorded in the set by means of this function. The set will record the value of the measurement in kilos and the date and time it is carried out.

- VI BLOOD ANALYSIS: There are several parameters for blood which are relevant to the patient's diagnosis. These are the following:

  - Cholesterol
  - HDL-C (high-density lipoprotein)
  - LDL-C (low-density lipoprotein)
  - Triglycerides
  - Fructosamine
  - Haemoglobin Alc.

When the patient carries out a blood analysis, these data may be entered into the system by the patient himself, by the laboratory which carries out the analyses or by the doctor who attends him.

The above-mentioned data are entered manually, so they may not be free from errors. Although the set will have mechanisms that help to validate the data entered, there will always be a margin of error due to the manual nature of the operation.

## 4.- STATISTICS

### TABLES AND GRAPHS

These show the evolution over time of the patient's analyses.

### GLUCOSE LEVEL

This allows the patient to take measurements of blood glucose level.

### BLOOD PRESSURE

This shows the evolution over time of the patient's blood pressure.

### HEIGHT

This shows the evolution over time of the patient's height.

### WEIGHT

This shows the evolution over time of the patient's weight.

### BLOOD ANALYSIS

This shows the evolution over time of the patient's blood analyses.

### CHOLESTEROL

This shows the evolution over time of cholesterol levels.

### HDL-C

This shows the evolution over time of HDL levels.

### LDL-C

This shows the evolution over time of LDL levels.

### TRIGLYCERIDES

This shows the evolution over time of triglyceride levels.

### FRUCTOSAMINE

This shows the evolution over time of fructosamine levels.

### HAEMOGLOBIN Alc.

This shows the evolution over time of haemoglobin levels.
The information that this function provides is very useful and serves to correctly interpret the patient's evolution under a specific treatment.
The analysis may be carried out in two different ways: by means of tables or graphs.
In table form, the statistical information regarding the parameter to be analysed will comprise all the entered measurements, although it will be limited by the screen space available on the set, so techniques of continuous display will be used to scroll through the data.
In graph form, only measurements in a given time interval will be displayed.
In the case of measurements of blood glucose levels, an indication of data concerning exercise and self-dosage that the patient has introduced will appear together with the same.

## 5.- CLINICAL REPORT

This shows the patient's last medical record.
The mobile terminal's computer allows the clinical report prepared by the doctor to be stored. This report is very useful for the patient, who may show it to any doctor that attends him. The report is entered and loaded into the system by means of a GSM communication.

## 6.- SOS

This carries out an emergency call to the doctor or the Control Centre.
In the event of an emergency the patient may quickly request the attention of his doctor by activating this function.
The emergency telephone number of the doctor or the Central Unit will be recorded on the set, so that when this function is pressed, the call will be automatic and no additional function will need to be pressed.

## 7. - INFORMATION

This selects and shows the patient's data and those of the doctor attending him.

### DOCTOR

This shows the data of the doctor attending the patient.

## PATIENT

This shows the data relating to the patient being treated.

## PATIENT SELECTION

This allows one of the patients who are registered to be selected.

## SYSTEM FUNCTIONS

This allows set parameters of interest to be displayed.

## PRINTING

This allows to print the stored data tables.
As regards the set, there is a series of data that may be useful, such as the available memory, diskette unit version, etc.. Although this information is not vital for the patient, it is very useful for maintaining the set.

## 8. - TELEPHONE

This allows communications to be carried out.

## VOICE TELEPHONE

This allows the system to be used as a normal call telephone.

## MESSAGE RECEPTION

This allows messages to be received through the Short Message Service.
By means of this function the patient may use the system in the same way as he would use the basic functions of a conventional GSM telephone terminal.
At all times calls received could be attended by following the on-screen instructions. Only when carrying out an analysis of blood glucose level will the incoming call function be de-activated, in which case it will be diverted to the mailbox GSM until the patient finishes the analysis.
These 8 functions have been conceived in an open environment, which allows more options to be added to the mobile terminal's computer in the future only by means of a programme change to the set, such as telephonic modem communications, downloading of data to a computer, etc.
This is possible thanks to the apparatus' graphic interface representation. The only exception to the new functions with respect to the eight access keys described is that they will have to be selected by scrolling through the screen using the cursor keys and executing the selected function with the ENTER key.

## Claims

1. A telematic patient monitoring system that:

   - Automatically analyses a specific physiological parameter indicating a specific disease, in a preferably portable peripheral unit (patient's terminal).
   - Records the said analysed data, together with other data associated with the time the measurement is taken, in a data base of records, in the memory of a computer contained in the patient's peripheral unit itself.
   - Sends the set of data recorded in the memory of the computer of the patient's peripheral unit, which includes the numerical value obtained from the parameter analysed, to a central unit for processing.
   - Monitors each patient individually or in a group in the said central unit and/or in conjunction with other peripheral units (laboratory and/or medical terminals).
   - Sends a command from the central unit to the patients' peripheral units and/or laboratory terminals and/or medical terminals, based on the result of the analysis of the set of data received from the patient's peripheral unit and its comparison, at least with the general data base of the said central unit.

2. A telematic patient monitoring system as claimed in claim 1 characterized in that communication between the central unit and the peripheral units, and vice versa, is by means of mobile cellular telephony.

3. A telematic patient monitoring system as claimed in claims 1 and 2 characterized in that in the patient's peripheral unit the recorded data constitute a resident history base of records that may be consulted by the patient himself, by the central unit and/or by other peripheral units.

4. A telematic patient monitoring system as claimed in any of the foregoing claims characterized in that the resident history base of records in the patient's peripheral unit may intervene in the statistical processing of the set of data received, at a given moment, from the patient himself, and which is carried out in the central unit.

5. A telematic patient monitoring system as claimed in any of the foregoing claims characterized in that the computer of the patient's peripheral unit is provided with a programme that compares the data received from the autoanalyzer, together with the associated data recorded at the time of analysis, with a set of reference values recorded for each patient, and issues a specific command or signal, both in the patient's peripheral unit itself and in the other units, including the central unit.

6. A telematic patient monitoring system as claimed in claim 5 characterized in that the set of reference values recorded in the computer of the patient's peripheral unit may be modified over time.

7. A telematic patient monitoring system as claimed in any of the foregoing claims characterized in that the command or signal sent from the central unit to the peripheral units may, once the set of data previously received are processed, consist of one or several of the following indications:

   • medication
   • diet
   • treatment
   • change in the sequence of controls to be carried out by the patient
   • levels of alarm associated with the patient's disease.

8. A telematic patient monitoring system as claimed in claim 7 characterized in that the commands or signals from the central unit are received in the patient's peripheral unit by telephone by means of messages in the computer by modem or directly by voice.

9. A telematic patient monitoring system as claimed in any of the foregoing claims characterized in that the physiological value analysed is the concentration in blood or urine of a biochemical parameter.

10. A telematic patient monitoring system as claimed in any of the foregoing claims characterized in that the biochemical parameter analysed is glucose and the patients monitored are diabetics.

11. A telematic patient monitoring system as claimed in any of the foregoing claims characterized in that the central unit comprises:

   Interconnected fault tolerant server computers
   Multi-area operating system
   Consultation terminals
   Local area network
   Data base
   Management, monitoring and control programmes
   Router communications and links with X.25 port or similar and configurable to different transmission speeds.
   Point-to-point communications and links at different transmission speeds, with different communication protocols and virtual switched circuits, connectable to different types of modems (including those providing access to digital communications via radio, such as GSM, DCS, PCN and the like).
Complete electrical energy supply system, with uninterruptable supply unit, connections and protections.
Communications components and links, with patients' terminals, doctors' terminals and laboratory terminals and whatever other terminals are necessary for the exchange of information and data.
Auxiliary components: printers, screens and the like.

12. A telematic patient monitoring system as claimed in any of the foregoing claims characterized in that the laboratory terminals comprise:

   Personal computers (PCs)
   Operating programmes for the personal computers
   Data bases for the personal computers
   Modems for connection to fixed or cellular telephone networks, whether analogue or digital.
   Fixed or portable cellular telephones, whether digital or analogue.
   Other components for interconnection with the Central Unit, patients' terminals and doctors' terminals.

13. A telematic patient monitoring system as claimed in any of the foregoing claims characterized in that the medical terminals comprise:

   Personal (PC) fixed or portable computers.
   Operating programmes for the computers.
   Data bases for the computers.
   Modems for connection to fixed or cellular telephone networks, whether analogue or digital.
   Fixed or portable cellular telephones, whether digital or analogue.
   Specific programmes for control and monitoring of patients' medical parameters, and for monitoring and dialogue with the Central Unit, laboratory terminals and between medical terminals.
   Communications, linking, control and monitoring programmes and apparatus of the Central Unit, patient terminals, laboratory terminals and other doctors' terminals.

14. A telematic patient monitoring system as claimed in any of the foregoing claims characterized in that the patient's mobile terminal comprises:

   * a personal computer of small size (of the type called personal notebook, palm-top, pocket calculator, ADP, etc.), including both hardware and software.

* a glucose-measuring autoanalyzer.
* a patient's digital or analog cellular telephone terminal (GSM, DCS, PCN, etc.), with its programmes and utilities.
* a controller (CPU) whose functions range from dialling the telephone number of the other units to capturing data from the glucose measuring device in order to store it subsequently in the personal computer and send it to the other units (Central Unit, laboratory terminals or medical terminals), which contains:

  • a printed circuit board.
  • a low-consumption CMOS microprocessor.
  • non-collateral RAM memory for data storage.
  • flash EPROM memory for storing the application programme.
  • DES cryptography module to ensure communications.
  • SMS digital apparatus for interconnection with the telephone terminal.

* a supply battery.
* a moulded body or housing in which the personal computer, telephone terminal and autoanalyzer are integrated.
* operating programmes for management and communication of the different components of the patient's terminal with each other and with the sub-systems of the central unit, laboratory terminals and medical terminals.

15. An apparatus, preferably portable, of small size, for self-measurement of physiological parameters that may be used as a patient's terminal in the telematic monitoring system referred to in claims 1 to 14, which comprises:

  * a personal computer of small size (of the type called personal notebook, palm-top, pocket calculator, ADP, etc.), including both hardware and software.
  * a glucose level autoanalyzer.
  * a patient's digital or analog cellular telephone terminal (GSM, DCS, PCN, etc.), with its programmes and utilities.
  * a controller (CPU) whose functions range from dialling the telephone number of the other units to capturing data from the glucose measuring device in order to store it subsequently in the personal computer and send it to the other units (Central Unit, laboratory terminals or medical terminals), which contains:

  • a printed circuit board.
  • a low-consumption CMOS microprocessor.
  • non-collateral RAM memory for data storage.
  • flash EPROM memory for storing the application programme.
  • DES cryptography module to ensure communications.
  • SMS digital apparatus for interconnection with the telephone terminal.

* a supply battery.
* a moulded body or housing in which the personal computer, telephone terminal and autoanalyzer are integrated.
* operating programmes for management and communication of the different components of the patient's terminal with each other and with the sub-systems of the central unit, laboratory terminals and medical terminals.

## INTERNATIONAL SEARCH REPORT

| International application No. |
|---|
| PCT/ES 98/00011 |

A.  CLASSIFICATION OF SUBJECT MATTER

  CIP6  A61B 5/00

According to International Patent Classification (IPC) or to both national classification and IPC

B.  FIELDS SEARCHED

Minimum documentation searched (classification system followed by classification symbols)

  CIP6  A61B, G06F

Documentation searched other than minimum documentation to the extent that such documents are included in the fields searched

Electronic data base consulted during the international search (name of data base and, where practicable, search terms used)

   EPODOC, WPI, PAJ

C.  DOCUMENTS CONSIDERED TO BE RELEVANT

| Category* | Citation of document, with indication, where appropriate, of the relevant passages | Relevant to claim No. |
|---|---|---|
| X | US 5544661 A (DAVIS et al) 13 August 1996 (13.08.96) Column 1, lines 43-52, column 1, line 64-column 2 line 15; column 2, line 57-column 3, line 22; column 4 lines 11-21; column 6, lines 36-41; column 7, lines 5-29, lines 46-50; column 8, lines 40-55; column 9, lines 15-53, lines 63-67; column 10, lines 5-10, lines 15-18; fig. 1,3-5,15 | 1,2,5-8,12,13 9,10,11,14,15 |
| Y | WO 9641288 A (E-SYSTEM) 19 December 1996 (19.12.96) Page 9, line 1-page 10, line 14; fig.1 | 11 |
| Y | US 473176 A (ALLEN,III) 15 March 1988 (15.03.88) Column 3, lines 43-63; column 4, lines 51-64; column 6, lines 28-68; fig. 2,4 | 9,10,14,15 |
| A | EP 0417944 A (NOKIA MOBILE PHONES) 20 March 1991 (20.03.91) Column 1, line 38-column 2, line 8 | 14,15 |

☐ Further documents are listed in the continuation of Box C.   ☒ See patent family annex.

* Special categories of cited documents:

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step when the document is taken alone

"Y"  document of particular relevance; the claimed invention cannot be considered to involve an inventive step when the document is combined with one or more other such documents, such combination being obvious to a person skilled in the art

"&"  document member of the same patent family

| Date of the actual completion of the international search | Date of mailing of the international search report |
|---|---|
| 16 March 1998 (16.03.98) | 26 March 1998 ( 26.03.98 ) |

| Name and mailing address of the ISA/ | Authorized officer |
|---|---|
| | |
| Facsimile No. | Telephone No. |

Form PCT/ISA/210 (second sheet) (July 1992)

## INTERNATIONAL SEARCH REPORT

International application No.

PCT/ES 98/00011

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | WO 9424929 A (HEALTHDYNE) 10 November 1994 (10.11.94)<br>Pag.6, line 6-pag. 8, line 2; pag. 12, line 18-<br>pag. 13, line 3; page 19, lines 14-31; pag. 20,<br>line 34-pag.21, line 6; pag. 27, lines 10-31; page 28,<br>lines 17-22; pag. 29, lines 7-37; page 31, line 20-<br>pag.32, line 30; page 33, lines 3-20; page 33, line 35-<br>pag. 34, line 6; fig. 1-4 | 1,7,9,10,12,13<br><br>2,11<br>14,15 |
| Y | WO 9508900 A (NOKIA TELECOMMUNICATIONS OY) 30 March<br>1995 (30.03.95) Pag.1, line 22- pag.3,line 2; pag.9<br>lines 16-24; claim 11; fig,1,2 | 2,11<br>12,15 |
| X<br>A | WO 9532480 A (ENACT PRODUCTS) 30 November 1995<br>(30.11.95) Pag.8, line 21-pag. 9, line 26; pag.13,<br>lines 13-26; fig.1-3 | 1,5,6,9,10<br>14,15 |
| A | WO 9425927 A (HEVER FOR LIFE FOR HEALTH FOR SPIRIT)<br>10 November 1994 (10.11.94) Pag.9, lines 24-28;<br>pag. 10, line 11-pag.11, line 8, pag.13, lines 14-24;<br>pag.16, lines 4-13; pag.17,line 1- pag.18, line 2;<br>pag.22, lines 20-28; pag.23, lines 10-23 | 9,11-13 |
| A | WO 9625877 A (BRIGHAM AND WOMEN'S HOSPITAL)<br>29 August 1996 (29.08.96) Pag.9, line 19-pag.12,<br>line 4; pag.18,line 10-pag.19, line 9; pag.52,line 16-<br>pag.53,line 8; pag.57, line 18-pag.58, line 5; pag.59,<br>lines 10-17; pag.60, line 8 - pag.61, line 2; claims 1,<br>6,15,16,29,30; fig 1 | 1,11-15 |

Form PCT/ISA/210 (continuation of second sheet) (July 1992)

## INTERNATIONAL SEARCH REPORT
Information on patent family members

| | International Application No |
|---|---|
| | PCT/ES 98/00011 |

| Patent document cited in search report | Publication date | Patent familiy member(s) | Publication date |
|---|---|---|---|
| US 5544661 A | 13.08.96 | NONE | |
| WO 9641288 A | 19.12.96 | AU 6252496 A | 30.12.96 |
| US 4731726 A | 15.03.88 | CA 1304135 A | 23.06.92 |
| EP 0417944 A | 20.03.91 | JP 3112751 A<br>AT 121035 T<br>DE 69018550 D<br>DE 69018550 T | 14.05.91<br>15.04.95<br>18.05.95<br>14.12.95 |
| WO 9424929 A | 10.11.94 | US 5558638 A<br>EP 0699046 A<br>AU 6779194 A | 24.09.96<br>06.03.96<br>21.11.94 |
| WO 9508900 A | 30.03.95 | JP 9505951 T<br>AU 678534 B<br>EP 0720806 A<br>AU 7658694 A<br>FI 934115 A,B,C<br>CN 1133666 A | 10.06.97<br>29.05.97<br>10.07.96<br>10.04.95<br>21.03.95<br>16.10.96 |
| WO 9532480 A | 30.11.95 | JP 10500598 T<br>US 5704366 A<br>US 5626144 A<br>US 5549117 A<br>EP 0765507 A<br>CA 2190283 A<br>AU 2646395 A | 20.01.98<br>06.01.98<br>06.05.97<br>27.08.96<br>02.04.97<br>30.11.95<br>18.12.95 |
| WO 9425927 A | 10.11.94 | US 5542420 A<br>CA 2161627 A<br>AU 7092294 A | 06.08.96<br>10.11.94<br>21.11.94 |
| WO 9625877 A | 29.08.96 | AU 5356996 A | 11.09.96 |

Form PCT/ISA/210 (patent family annex) (July 1992)